# EUROPEAN PATENT APPLICATION

(11) **EP 2 916 132 A1**
(43) Date of publication of application: **09.09.2015**
(21) Application number: 14157944.1
(22) Date of filing: 05.03.2014
(51) Int. Cl.: G01N 33/548

(54) **Method for photo-immobilizing biomolecules on a cellulose carrier**

(71) Applicant: Commissariat à l'Énergie Atomique et aux Énergies Alternatives, 75015 Paris (FR)
(72) Inventor: Credou, Julie, 94230 Cachan (FR); Berthelot, Thomas, 91940 Les Ulis (FR)
(74) Representative: Icosa

(57) **Abstract**

The present invention relates to the immobilization of biomolecules on a cellulose carrier by irradiating the biomolecule-impregnated carrier with a light of a wavelength of at least 340 nm.

## Description

### FIELD OF INVENTION

The present invention relates to the immobilization of biomolecules on a cellulose carrier by irradiating the biomolecule-impregnated carrier with a light of a wavelength of at least 340 nm.

### BACKGROUND OF INVENTION

In various domains such as clinical diagnosis, drug screening, food quality control, environmental monitoring, there is a need to easily and rapidly detect biomolecules that are free in solution or attached to the surface of a cell, a virus or a *bacteria.* The biomolecules to be detected are essentially composed of proteins or peptides, such as enzymes, antibodies, receptors, transcription factors, hormones, of nucleic acids or oligonucleotides or of carbohydrates and the like. In the field of clinical diagnosis, the identification of proteins in blood, urine or biopsy samples can for instance be useful for detecting bacterial or viral infections, cancers, diabetes, hormonal imbalance or pregnancy. Detecting pathogenic microorganisms may also be of primary importance for environmental purposes, and may be implemented for controlling the quality of the water and/or food.

Several methods have been developed for manufacturing biosensors, biochips, microarray and other immunoassay devices. It may, for instance, be noticed that blood glucose sensors, pregnancy tests, or urine test strips are among the most commonly distributed devices for identifying biomolecules.

The preparation of efficient bioassay devices, and more specifically of immunoassay devices, requires the robust immobilization of a large number of biomolecules of interest on a carrier. As a result of its improved capacity to immobilize proteins, and more specifically antibodies, by electrostatic interaction with the nitro functions displayed on its surface, nitrocellulose constitutes the most commonly used carrier material for preparing immunochromatographic devices. Nevertheless, nitrocellulose is an expensive, fragile and inflammable material, which was furthermore shown to be incompatible with newly developed multiplex biosensors such as lab-on-paper devices, microfluidic paper analytical devices (µPADs), or other paper-based analytical devices. Moreover, some agents such as spores and some *bacteria* may have difficulty in migrating along nitrocellulose. For these reasons, nitrocellulose is thus progressively replaced by cellulose.

Cellulose is an affordable biopolymer, which is also biocompatible, biodegradable and easily available. Cellulose is of specific interest since it exhibits wicking properties allowing biomolecules in solution to migrate by capillarity without needing any external power sources. It is further available in a broad range of thickness and possesses well-defined pore sizes, is easy to store and safely disposable.

Several methods for immobilizing biomolecules on cellulose supports are known in the art, which may be classified in three major families: (i) physical methods, wherein the biomolecule is retained on the support through physical forces such as electrostatic, Van der Waals, hydrophobic interactions and the like; (ii) biological or biochemical methods wherein the biomolecule is linked to the support through biochemical affinity between two components *(e.g.* metal/ligand, ligand/protein, protein/antibody, *etc.*); and (iii) chemical methods, wherein covalent bonds maintain the biomolecule on the support. Nevertheless, each of these methods also display specific drawbacks.

Physical methods may be implemented through simple, rapid and cost-saving procedures, and advantageously limit the necessity for modifying the biomolecule or its support. Nevertheless, the weak and non-permanent interaction maintaining the biomolecule on the support also represent a major drawback of these methods, since biomolecules are progressively torn out, thus triggering a loss of activity of the corresponding biosensor.

Biological methods advantageously allow the biomolecules to be immobilized in a specific orientation, through reversible non-covalent, whereas strong and specific, interactions with the support. Nevertheless, these methods require complex and expensive procedures wherein the biomolecule and/or the support are modified for introducing a binding conjugate or a binding domain therein.

Finally, chemical methods provide a strong, stable and permanent coupling of the biomolecule to its support. The thus-conceived biosensors are robust and provide reproducible results. Nevertheless, the chemical treatments performed may modify and alter the structure and/or the activity of the biomolecules. The resulting biosensors, while reusable, may thus lack sensitivity as a consequence of biomolecule alteration.

Among the known covalent coupling techniques, photo-immobilization is probably the simpler and the faster to be implemented in a process for preparing bioassay devices and more specifically immunoassay devices: the support is generally coated with a photoreactive compound and the biomolecule of interest is covalently affixed to the support through photoactivation. Considering that short-wave UV (ultraviolet) light (*i.e.* 100 nm - 400 nm) is known to alter biomolecules, photoimmobilization is then regularly performed under long-wave UV light (340 nm - 400 nm) or visible light (400 nm - 800 nm) (Viel et al., 2013, Langmuir 29:2075-2082; Volland *et al.,* 2004, 34: 737-752). Nevertheless, all the photoimmobilization methods described so far require to use a photoreactive coupling intermediate, and further require the functionalization of cellulose through harsh conditions, in organic solvents, or with highly toxic reagents or side products.

There is therefore an ongoing need for cost-saving and rapid methods allowing bioassay devices, and more particularly immunoassay devices, to be prepared by immobilizing biomolecules on cellulose carriers through robust and sustainable binding. There is indeed a long-felt need for immobilization methods displaying a limited number of steps, allowing to save significant amounts of reactants solvents or adjuvants, and at the same time capable to preserve the activity of the biomolecules of interest through the use of mild-coupling reaction conditions.

This need is now fulfilled by the process of the present invention. The present inventors have indeed surprisingly discovered that biomolecules such as proteins (e.g. antibodies) can advantageously be photo-immobilized on a cellulose carrier in absence of any photocoupling intermediate, upon exposure to a light of a wavelength of at least 340 nm. The process of the invention thus surprisingly and advantageously allows biomolecules to be immobilized on a cellulose carrier with a coupling efficiency similar to that observed for nitrocellulose, and unexpectedly preserves the said biomolecules from the loss of activity resulting from known chemically-mediated photoimmobilization methods. The process of the invention thus appears to be faster, cost-saving and environmentally-friendly, since non-functionalized cellulose is an affordable and easily available biodegradable biopolymer.

### SUMMARY

This invention thus relates to a process for immobilizing biomolecules on a carrier comprising the steps of (i) impregnating the carrier with a solution containing the said biomolecules; and (ii) irradiating the impregnated carrier resulting from step (i) with a light of a wavelength of at least 340 nm; wherein said carrier is composed of cellulose.

In a particular embodiment of the invention, the said carrier is preferably composed of non-functionalized and/or chemical-free cellulose.

In a specific embodiment, the process of the invention further comprises a step of drying said impregnated carrier after step (i) and before step (ii). In particular embodiment, the process further comprises at least one step of washing the irradiated support resulting from step (ii).

In a particular embodiment, the irradiation light used for implementing the process of the invention has a wavelength of from 340 nm to 800 nm, preferably of from 340 nm to 400 nm, preferably of from 400 nm to 800 nm, and is preferably of about 365 nm.

In a particular embodiment, the irradiation step (ii) is performed with a photoenergy of from 1 mJ/cm² to 500 J/cm², preferably of from 1 J/cm² to 80 J/cm², and is preferably of about 10 J/cm².

In a particular embodiment, the irradiation step (ii) is performed for a period of at least 1 second, preferably from 16 to 1280 minutes, preferably of about 160 minutes.

In a particular embodiment, the carrier for use in the process of the invention is selected in the form of a bead, well, sheet, powder, stick, plate, strip or as a tube, and is preferably in the form of a sheet.

In a particular embodiment, the biomolecule is not functionalized and is selected from the group consisting of proteins or peptides, such as antibodies, antigens, enzymes, transcription factors, protein domains or binding proteins. In a particular embodiment, the biomolecule is displayed on the surface of a *bacteria,* a virus or a micro-organism or is free in solution.

In a particular embodiment, the said biomolecule is solubilized in a solvent selected in the group consisting of: water, phosphate buffer, carbonate buffer, borate buffer, HEPES (4-(2-hydroxyethyl)-1-piperazineethanesulfonic acid) buffer, MES (2-(N-morpholino)ethanesulfonic acid) buffer or any other aqueous biological buffer.

In a particular embodiment of the process of the invention, the washing step is performed with a buffer selected from the group consisting of water, phosphate buffer, carbonate buffer, borate buffer, HEPES buffer, MES buffer or any other aqueous biological buffer, and further optionally comprises salts and/or detergent.

In a particular embodiment, the process for immobilizing biomolecules on a carrier according to the invention comprises the steps of (i) impregnating the carrier with a solution containing the said biomolecules; (ii) drying the impregnated carrier resulting of step (i); and (iii) irradiating the dried impregnated carrier resulting from step (ii) with a light of a wavelength of at least 340 nm and with a photoenergy of at least 1 mJ/cm², preferably of at least 1 J/cm², and preferably of about 10 J/cm².

The present invention also concerns a grafted cellulose carrier obtained by the process of the invention, wherein said cellulose carrier comprises biomolecules immobilized thereonto.

The present invention also concerns a bioassay device, preferably an immunoassay device, comprising at least one cellulose carrier of the invention, wherein said bioassay device is preferably an immunochromatographic strip or an immunochromatographic multiplex system.

The present invention also concerns the use of at least one cellulose carrier or of a bioassay device according to the invention for diagnosis, affinity chromatography, proteomics, genomics and/or drug screening.

The present invention also concerns a bioassay kit comprising at least one cellulose carrier or at least one bioassay device according to the invention.

### DETAILED DESCRIPTION

This invention therefore relates to a surprising and advantageous process allowing biomolecules to be immobilized on a cellulose carrier and comprising the steps of (i) impregnating the carrier with a solution containing the said biomolecules; and (ii) irradiating the impregnated carrier resulting from step (i) with a light of a wavelength of at least 340 nm.

Within the present invention, by "impregnating", it is meant that biomolecules, in suspension in a solvent, are dispensed onto the cellulose carrier by any method known in the art, such that the said biomolecules in solution are placed into contact with the cellulose fibers of the carrier. The biomolecule solution may thus be dispensed onto the carrier under the form of drops, or droplets, such as for instance those formed by a printing system or by printing techniques such as silkscreen printing, inkjet printing, spraying, spin coating, and the like. The carrier may alternatively be immerged in a solution comprising the said biomolecules.

Within the present invention, by "cellulose", it is meant a polysaccharide of formula (a) (see below), consisting of a linear chain of several hundred to over ten thousand β(1-4) linked D-glucose units.

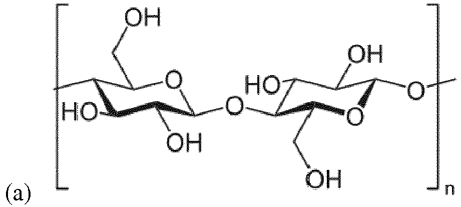

Cellulose for use within the present invention may be obtained from any origin, such as green plants, algae, oomycetes or *bacteria.* Cellulose for use in the present invention is non-functionalized, *i.e.* it has not been treated chemically for generating reactive moieties, nor coated with chemically- and/or photo-reactive intermediates. In a preferred embodiment, cellulose for use in the present invention is pure cellulose paper. Depending on the destination of the grafted cellulose carrier prepared according to the process of the present invention, the density and/or shape of the cellulose carrier may vary. Shapes suitable for the grafted cellulose carrier of the invention include but are not limited to: a bead, a well, a sheet, a powder, a stick, a plate, a strip, a tube, as well as any 3D shape alternative thereof. Cellulose provided under the form of a sheet is also known in the art under the term "paper".

Within the present invention, by "irradiating", it is meant subjecting the cellulose carrier impregnated with the solution of biomolecules to a light having a wavelength of at least 340 nm. In a preferred embodiment, the light used for irradiating the impregnated cellulose carrier has a wavelength of from 340 nm to 800 nm (long-scale UV). In another embodiment, the light used for irradiating the impregnated cellulose carrier has a wavelength of from 400 nm to 800 nm. In a preferred embodiment, the light used has a wavelength of about 365 nm.

Within the process of the invention, irradiation is conducted for a period of time and with a wavelength that are suitable for subjecting the impregnated cellulose carrier to a photoenergy of from 1 mJ/cm² to 500 J/cm², preferably of from 1 J/cm² to 80 J/cm². In a preferred embodiment, irradiation is conducted for a period of time and with a wavelength that are suitable for subjecting the impregnated cellulose carrier to a photoenergy of about 10 J/cm².

Within the process of the invention, irradiation is conducted for a period of time of at least 1 second, preferably from 16 to 1280 minutes, preferably of about 160 minutes.

Within the present invention, by "immobilized", it is meant that moieties of the biomolecules of interest present in the solution used for impregnating the cellulose carrier are covalently coupled to moieties of the cellulose molecules, by co-chemical reaction triggered by irradiation. As a result of the process of the invention, the biomolecules of interest are thus covalently coupled to the cellulose carrier, while having preserved 80%, preferably 90%, preferably 99% and more preferably 100% of their biological activity.

Within the present invention, by "biomolecule", it is meant any biological molecule selected from the group consisting of proteins or peptides, such as antibodies, antigens, enzymes, transcription factors, protein domains or binding proteins. In a preferred embodiment, the biomolecules for use in the present invention are antibodies and proteins. In a preferred embodiment, the biomolecules for use in the present invention are native biomolecules, *i.e.* they are not modified either through chemical processes or by genetic engineering for improving their cellulose binding properties. In another preferred embodiment, the biomolecules for use in the present invention are modified, for example through the addition of a labelling system. In a preferred embodiment, the biomolecules of interest may correspond to biomolecules involved into biological membranes such as transmembrane proteins or antigenic proteins or peptides displayed on a biological membrane. In a particular embodiment of the present invention, the biomolecules of interest may be involved in the biological membrane of a cell, in the outer membrane or in the cell wall of a *bacteria* or a virus. In a preferred embodiment, by a solution of "biomolecules", it is also meant a solution wherein the biomolecules of interest are provided on the membrane, cell wall or envelope of a cell, a *bacteria* or a virus, together with the said cell, *bacteria* or virus. In another embodiment, the biomolecules for use in the present invention correspond to biomolecules which are not excreted by cells such as non-excreted proteins. In another embodiment, the biomolecules for use in the present invention are free in solution, and may have been either purified from a biological organism or synthesized *in vitro.* For use in the method of the invention, the biomolecules are solubilized in a solvent including but not limited to: water, water for injection, phosphate buffer, carbonate buffer, borate buffer, HEPES buffer, MES buffer or any other aqueous biological buffer.

Within the present invention, by "drying", it is meant evaporating the solvent wherein the biomolecules of interest are solubilized, thus allowing the corresponding biomolecules to be concentrated on or around the cellulose fibers of the carrier. In a preferred embodiment, the cellulose carrier impregnated with the solution containing the biomolecules of interest is dried before the irradiation step. Drying may be performed by any mean known in the art, and in particular in subjecting the impregnated paper to a source of heating, such as for instance, a ventilated oven.

In a particular embodiment of the present invention, a drying step is implemented before the irradiation step when said irradiation step (iii) is performed with a photoenergy of at least 1 mJ/cm², preferably of at least 1 J/cm², and preferably of about 10 J/cm².

Within the present invention, by "washing", it is meant applying a liquid on the irradiated cellulose carrier (*i.e.* after the irradiation step) for removing any unbound biomolecule. According to the process of the invention, the washing step may be performed with any solvent which does not alter the structure or the biological activity of the immobilized biomolecules and which does not alter the cellulose carrier. Preferred washing solutions for the use in the present invention include but are not limited to: water, water for injection, phosphate buffer, carbonate buffer, borate buffer, HEPES buffer, MES buffer or any other aqueous biological buffer, optionally enriched with salts and/or detergent.

Another object of the present invention concerns a grafted cellulose carrier obtained by the process of the invention wherein said cellulose carrier comprises biomolecules immobilized thereonto.

Within the present invention, by "grafted cellulose carrier", it is meant a cellulose carrier wherein biomolecules of interest were immobilized through the process of the invention, *i.e*. though irradiation at a wavelength of at least 340 nm. As a result of the process of the invention, the said grafted cellulose carrier is thus composed of biomolecules of interest which are covalently linked to a cellulose carrier absent any chemical intermediate or adjuvant.

Another object of the present invention concerns a bioassay device, preferably an immunoassay device, such as for instance an immunochromatographic strip or an immunochromatographic multiplex system.

Within the present invention, by "bioassay device", it is meant any device intended for detecting and/or quantifying biological or non-biological compounds, objects or organisms, including at least one grafted cellulose carrier of the invention.

Within the present invention, by "immunoassay device", it is meant any device intended for immunodetection or immunoquantification purposes including at least one grafted cellulose carrier of the invention.

Within the present invention, by "immunochromatographic strip", it is meant a device composed of a loading area, a detection area and an absorbent pad, the whole being affixed onto a plastic carrier. The detection area is formed by grafted cellulose carriers of the invention. Migration is supported by two migration areas, surrounding the detection area, that are capable to trigger the sample to be tested by adsorption to the detection area.

Within the present invention, by "immunochromatographic multiplex system", it is meant a system generally composed of hydrophilic paper channels, delimited by hydrophobic plastic stencils, such as those disclosed in patent applications filed under Nos. FR1161722 and FR1260083. The several levels of the system are stacked and connected together for forming a 3D fluidic system. A sample introduced at the top of the system is divided in as many aliquots as the number of channels, and is thus allowed to contact various biomolecules of interest immobilized on cellulose carriers according to the invention, and displayed in separated assay areas. An immunochromatographic multiplex system for instance allows different antigens to be detected within a same sample, using several cellulose carriers grafted with different antibodies.

Another object of the present invention concerns the use of at least one cellulose carrier according to the invention or of an immunoassay device according to the invention for diagnosis, affinity chromatography, proteomics, genomics and/or drug screening.

### BRIEF DESCRIPTION OF THE DRAWINGS

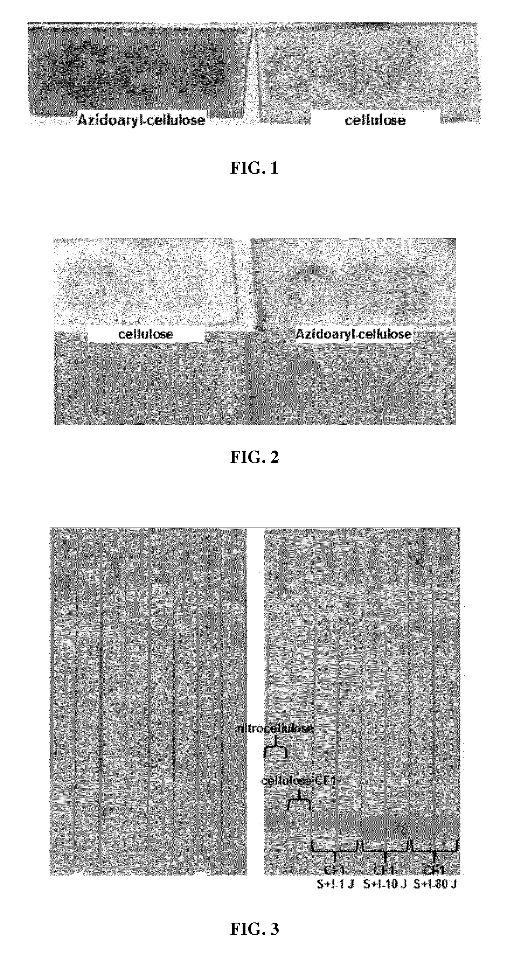

**Figure 1** is a photograph showing the localized immobilization of labelled antibodies through a mask. The localized immobilization is observed on the left on the functionalized cellulose (expected result), as well as on the right on pristine cellulose (unexpected).
**Figure 2** is a photograph showing the localized immobilization of murine antibodies. Murine antibodies immobilized on functionalized cellulose (on the right) or on pristine cellulose (on the left), are revealed by gold-labelled goat-anti-murine antibodies after saturation of the membrane with gelatin.
**Figure 3** is a photograph showing the influence of irradiation energy on antibody immobilization. OVA1 antibodies immobilized on nitrocellulose or cellulose, after optional drying (S) of the membrane, and irradiation (I) at 1 J/cm², 10 J/cm² or 80 J/cm², are revealed by gold-labelled OVA35 antibodies. In absence of OVA antigen (left panel), no signal is detected. In presence of OVA antigen (right panel), performances of nitrocellulose are reached for an irradiation energy of 10 J/cm². The results corresponding to 2 different samples are shown.

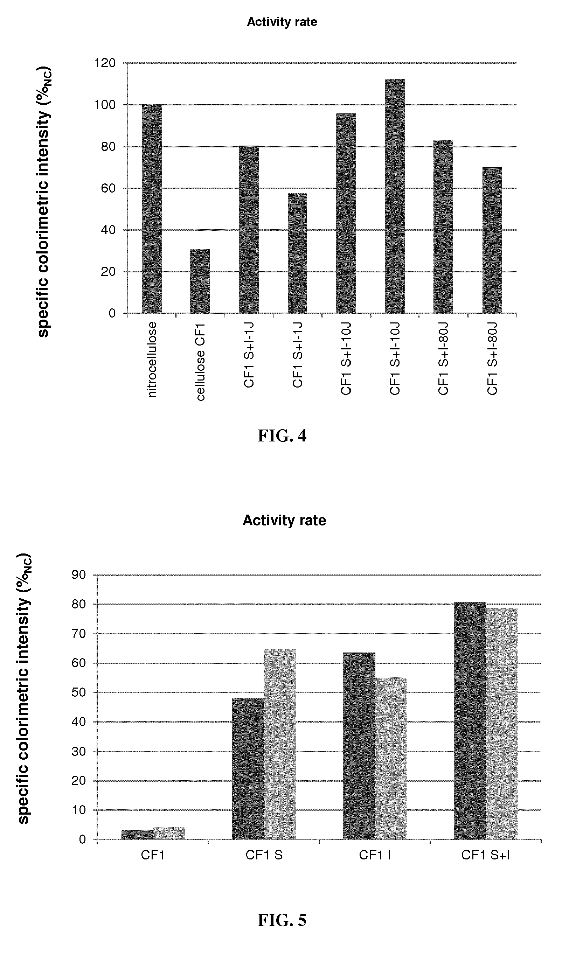

**Figure 4** is a histogram showing the specific colorimetric intensity obtained for lanes of the right panel of figure 3. Fig. 4 shows that the activity rate of antibodies adsorbed on nitrocellulose is reached on cellulose after drying and irradiating with 10 J/cm². The results corresponding to 2 different samples are shown, excepting for controls (nitrocellulose and cellulose).
**Figure 5** is a histogram showing the activity rate of antibodies immobilized on cellulose, after drying (S), irradiation (I) or drying and irradiation (S+I). The results of 2 different samples are presented for each condition.

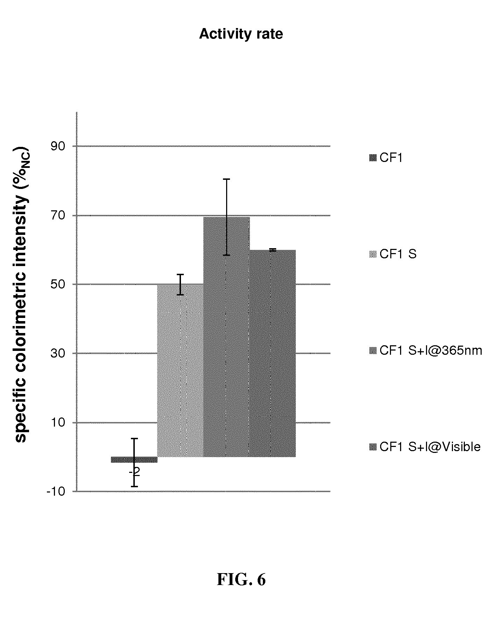

**Figure 6** is a histogram showing the activity rate of antibodies immobilized on cellulose after drying (S), 2^{nd} column from the left, drying and irradiating at 365 nm (S+I@365), 3^{rd} column from left, and drying and irradiating with visible light (S+I@visible), 4^{th} column from left.

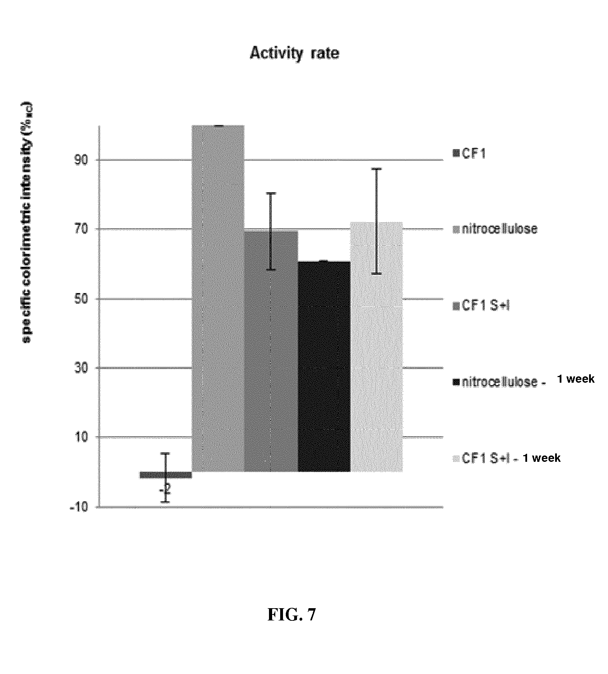

**Figure 7** is a histogram showing the activity rate of antibodies immobilized on cellulose without treatment (CF1, 1^{st} lane from left), after drying and irradiating (3^{rd} lane from left), or after drying and irradiating, then leaving in oven for 1 week at 40°C (5^{th} lane from left). Results are compared to the activity rate measured after immobilization on nitrocellulose (2^{nd} lane from left), or on nitrocellulose after 1 week in oven at 40°C (4^{th} lane from left).

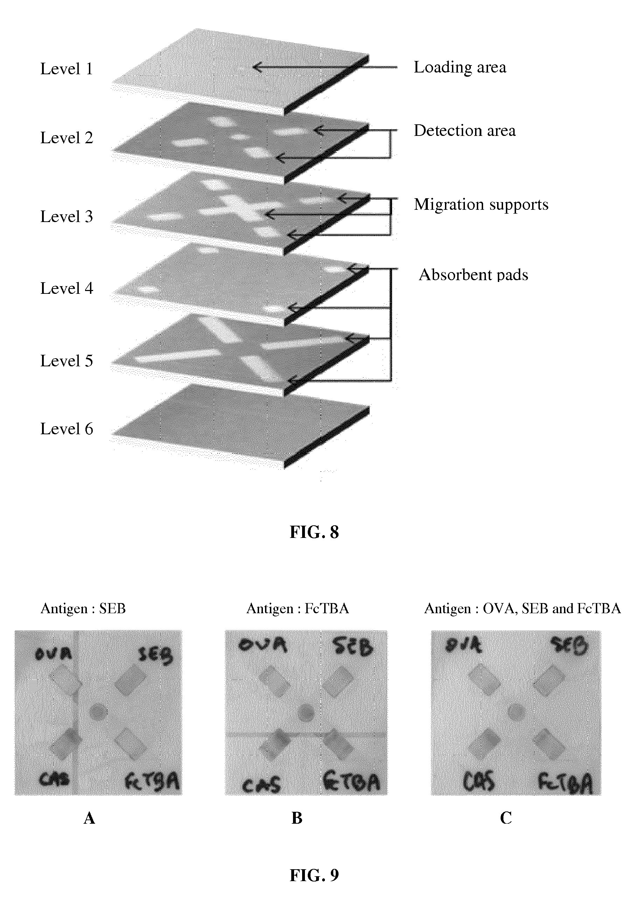

**Figure 8** is a drawing showing the general structure of an immunochromatographic assay multiplex. Level 1 corresponds to the loading area. Level 2 shows the detection areas. The sample introduced on the top of the multiplex is divided into 4 aliquots directed towards the 4 assay areas of the multiplex. Papers grafted with different antibodies are introduced in the various assay areas. Level 3 shows the migration supports. Levels 4 and 5 show the absorbent pads. Level 6 shows the supporting base.
**Figure 9** is a photograph showing the results of immunochromatographic assays conducted on multiplex systems. For each antigen tested, the coloration of the specific area where the corresponding specific antibody has been introduced is observed.

### EXAMPLES

The present invention is further illustrated by the following examples.

### Example 1: Localized immobilization of labelled antibodies through a mask (photolithography)

A solution of anti-murine goat antibodies, labelled with colloidal gold according to the standard method (Credou et al., 2013, J. Mater Chem. B, 1: 3277-3286) is diluted three times then poured on a 2 cm²-cellulose sheet (1 x 2cm), at a rate of 20 µL/cm². Arylazide-functionalized cellulose is compared to pristine cellulose. Drying is deliberately omitted for avoiding important background noise resulting from the adsorption of gold particles. A mask is placed on the antibody-impregnated membrane and the system is irradiated for 80 minutes (at 5 J/cm²). After mask removal, samples are rinsed overnight with a phosphate buffer. Surprisingly, localized immobilization of antibodies is observed not only on the functionalized paper (expected result) but also on the pristine paper (unexpected result) (see fig. 1).

### Example 2: Localized immobilization of murine antibodies

Simple murine antibodies are immobilized for ensuring that the selective photoimmobilization observed in Example 1 does not result from colloidal gold particles interference. Murine antibodies, provided in solution at a concentration of 1mg/mL in potassium phosphate buffer 0.1M, pH 7.4, are immobilized according to the process described in example 1. Membranes are saturated with gelatin, then the grafted antibodies are detected with anti-murine goat antibodies labelled with colloidal gold.

Antibody immobilization is observed on both functionalized and pristine papers (see fig. 2).

### Example 3: Influence of irradiation energy

Anti-ovalbumin OVA1 antibodies are poured on CF1 cellulose sheets, and further concentrated by drying the impregnated paper (S). The system is then irradiated (I) at 365 nm for various times, corresponding to different energy levels: 16 min (about 1 J/cm²), 2h40 (about 10 J/cm²) and 21h20 (about 80 J/cm²). Finally, paper is rinsed 3 times for 5 minutes with phosphate buffer.

As can be seen in fig. 3 and 4, performances of nitrocellulose are reached with an irradiation energy of 10 J/cm², for both grafting rate and activity rate.

### Example 4: Influence of drying upon short irradiation time

OVA1 antibodies are poured onto CF1 cellulose sheets. The impregnated papers are either dried for concentrating the antibodies (S) or left undried. Some dried and non-dried impregnated papers are then irradiated (I) at 365 nm for 1- min (about 1 J). Papers are rinsed with 3 successive baths (5 min each) in phosphate buffer. Grafting rate and activity rate are assessed.

Results indicate that drying appears to be required for short irradiation times (data not shown). Otherwise, antibodies remain in solution, too far away from fibers for ensuring a strong immobilization.

### Example 5: Influence of drying upon long irradiation time

OVA1 antibodies are poured onto CF1 cellulose sheets. The impregnated papers are either dried for concentrating the antibodies (S) or left undried. Some dried and non-dried impregnated papers are then irradiated (I) at 365 nm for 2h40 (about 10 J/cm²). Papers are rinsed overnight with phosphate buffer enriched in salts and detergent. Grafting rate and activity rate are assessed.

As can be seen in fig. 5, drying appears to be beneficial, but might be omitted for long irradiation times. Indeed, for long irradiation times, drying occurs naturally in the course of irradiation thereby allowing antibodies to get closer to the fibers.

### Example 6: Influence of the rinsing solution

OVA1 antibodies are poured onto CF1 cellulose sheets. The impregnated papers are either dried for concentrating the antibodies (S) or left undried. Dried impregnated papers are then irradiated (I) at 365 nm for 2h40 (about 10 J/cm²). Papers are rinsed either by 3 successive baths (5 min each) of phosphate buffer (P), or overnight with phosphate buffer enriched in salts and detergent (S/T). Grafting rate and activity rate are assessed.

Results indicate that extensive rinsing (e.g. overnight) with a phosphate buffer enriched in salts and detergent allows maintaining on the surface only molecules that are strongly immobilized (antibody adsorption is indeed reduced) (data not shown). The resulting signal appears to be slightly weaker, but results appear to be more reproducible.

### Example 7: Influence of paper nature

OVA1 antibodies are poured onto CF1, Chr1 or Xerox cellulose sheets. The impregnated papers are either dried for concentrating the antibodies (S) or left undried. Dried impregnated papers are then irradiated (I) at 365 nm for 2h40 (about 10 J/cm²). Papers are rinsed extensively with phosphate buffer enriched in salts and detergent. Grafting rate and activity rate are assessed.

Results indicate that the process of the invention allows the observed signal to be increased independently from the nature of the paper, with respect to adsorption alone (data not shown). The Xerox paper is treated for being hydrophobic: the antibody solution is thus hindered to penetrate between the fibers, thereby explaining a lower grafting rate. The process of the invention thus allows a larger quantity of functional antibodies to be strongly immobilized on any type of cellulose.

### Example 8: Influence of the wavelength

OVA1 antibodies are poured onto CF1 cellulose sheets. The impregnated papers are either dried for concentrating the antibodies (S) or left undried. Dried impregnated papers are then either irradiated (I@365) at 365 nm for 2h40 (about 10 J/cm²), irradiated under visible light for 2h40 (I@visible) or left unirradiated. Papers are rinsed extensively with phosphate buffer enriched in salts and detergent. Grafting rate and activity rate are assessed.

As can be seen in fig. 6, irradiation under visible light only slightly improves the grafting rate when compared to simple drying. Nevertheless, the corresponding activity rate appears to be slightly improved. Irradiation under visible light thus provides a better grafting than mere drying. Further, irradiation at 365 nm (more energetic) provides a better grafting than irradiation under visible light.

### Example 9: Influence of ageing

OVA1 antibodies are poured onto CF1 cellulose or on nitrocellulose sheets. The impregnated CF1 papers are dried for concentrating the antibodies (S) or left undried. Dried impregnated papers are then irradiated (I) at 365 nm for 2h40 (about 10 J/cm²). Papers are rinsed extensively with phosphate buffer enriched in salts and detergent. Grafting rate and activity rate are assessed. Nitrocellulose sheets are impregnated by the biomolecule solution and this system is left to incubate for Ih at room temperature.

Immunochromatographic tests are performed immediately after assembling of strips. Other strips, prepared previously and stored in oven 7 days at 40°C are used for assessing the effects of ageing.

As can be seen in fig. 7, ageing of nitrocellulose results in a decreased recognition of the grafted antibodies by the goat-anti-mouse antibody, as well as in a reduced biological activity. This phenomenon may be explained by the degradation of immobilized antibodies.

As regards cellulose, signal variability increases together with ageing. Recognition by goat-anti-mouse antibody is decreased, and may again result from the degradation of immobilized antibodies. Nevertheless, the observed decrease is less important than for nitrocellulose. Further, activity rate remains constant (when standard deviations are considered). These observations may be explained by the fact that antibodies grafted on cellulose through the method of the invention are less degraded at their binding site, or by the fact that they are "buried" in the paper, whereas antibodies are only displayed on the surface of nitrocellulose. Cellulose membranes according to the process of the present invention thus appear to be more resistant to ageing than nitrocellulose ones, and are therefore more suitable for use after storage.

### Example 10: Localized immobilization of labelled antibodies

Photolithography consists in transferring an image displayed on a mask towards a substrate through photochemical or photoactivated reactions. The process according to the present invention now allows photolithography to be performed on cellulose sheets. Opaque plastic maskings used in the present experiments have been designed and prepared with a laser plotter LaserPro Spirit (GCC Laser Pro, New Taipei City, Taiwan), and the software CorelDRAW Graphics Suite (Corel Corporation, Ottawa, Canada).

Probe antibodies labelled with colloidal gold were immobilized through a mask for allowing grafting of antibodies by photolithography to be observed directly, and for evaluating the signal/background *ratio.*

A solution of colloidal-gold-labelled goat-anti-mouse antibodies prepared accordingly to known methods (Khreich et al., Analytical Biochemistry 377 (2008) 182-188) is diluted three times, then poured onto a 2 cm² cellulose sheet (1x2 cm), at a rate of 20 µl/cm². Drying is intentionally omitted for avoiding important background levels resulting from the adsorption of gold particles. A mask is then placed on the antibody-impregnated membrane, and the system is irradiated for 80 minutes (about 5 J/cm²). After removal of the mask, samples are rinsed overnight with a phosphate buffer enriched in salts and detergent.

Gelatin (at a concentration of 1mg/mL in potassium phosphate buffer 0.1M, pH 7.4) is immobilized according to the same procedure, and is used as negative control. The immobilization rate of labelled antibodies is measured by assessing the differences between the signal obtained with the paper coated with labelled antibodies, and the signal obtained with the gelatin-coated paper.

Results indicate that a selective photoimmobilization of the colloidal-gold-labelled antibody is observed according to the design of the used mask (data not shown).

### Example 11: Localized immobilization of simple murine antibodies

Simple murine antibodies were immobilized for ensuring that the selective photoimmobilization observed in example 10 was not resulting from the mere binding of colloidal gold particles. The antibodies (in solution at a concentration of 1mg/mL in potassium phosphate buffer 0.1M, pH 7.4) were immobilized according to the general method used in previous examples. Membranes are then saturated with gelatin, and immobilized antibodies are detected with a colloidal-gold-labelled goat-anti-mouse antibody, similarly to the procedure previously implemented for evaluating the grafting rate.

Results indicate that the resulting photolithographic image matches with the selective immobilization of antibodies obtained by partial irradiation of the cellulose substrate (data not shown).

### Example 12: Immobilization of labelled antibodies

In order to assess the strength of grafting in the course of the photolithographic process, colloidal-gold-labelled antibodies were immobilized according to the general procedure implemented in previous examples. Following rinsing, colorimetric intensity is first measured. The antibody-grafted paper is then immersed in a solution of phosphate buffer enriched in salts and detergent and subjected to ultrasonic treatment for 20 minutes. Colorimetric intensity is measured again.

Results indicate that the colorimetric intensity measured after the ultrasonic treatment amounts to about 99% of the first intensity measured (data not shown). Considering that the observed signal decrease is comprised within the measuring error deviation, it can therefore be considered as non-significant. In conclusion, the grafting resulting from the process of the invention is thus very strong, if not covalent.

### Example 13: Immobilization of various antibodies

A multiplex system is generally composed of hydrophilic paper channels, delimited by hydrophobic plastic partition walls. The several levels of the system are stacked and connected by double-faced tape for forming a 3D fluidic system (see fig. 8). The sample introduced at the top of the dispositive is divided in 4 aliquots, directed towards the 4 parallel assay areas of the dispositive. Papers grafted with different antibodies are introduced in the various assay areas, and thus allow different antigens to be detected within a same sample.

In the present experiment, the introduced sample is composed of mixed antigens and corresponding tracers. 3 out of the 4 assay areas are used for detecting antigens within the sample, while the last one is used as a control area for assessing the presence of tracers within the migrating system. The control area is thus grafted with goat-anti-mouse antibodies. The antigen used are ovalbumin (OVA), staphylococcus enterotoxin B (SEB) and a fragment of botulinum toxin A (Fc-TBA). The solid phase/antigen/tracer systems used are : anti-OVA antibody (OVA1)/OVA/colloidal-gold-labelled anti-OVA antibody (OVA35), anti-SEB antibody (SEB27)/SEB/colloidal-gold-labelled anti-SEB antibody (SEB26) and anti-Fc-TBA antibody (TBA11)/Fc-TBA/ colloidal-gold-labelled anti-Fc-TBA antibody (TBA4). As described previously for measuring activity rates, antigen detection is performed by immunosandwich detection.

Paper channels and plastic position walls were cut using a laser plotter. Antibody grafted membranes were prepared similarly to the method previously described for preparing strips, *i.e.* through grafting, rinsing and saturating the membrane. Loading and migration areas were saturated with gelatin.

The various antibodies were therefore poured onto CF1 cellulose sheets, then concentrated by drying (S) of the impregnated paper. The resulting system was irradiated (I) at 365 nm for 2h40 (about 10 J/cm²). Paper was extensively rinsed with phosphate buffer enriched in salts and detergent.

The tested solution comprised the 3 tracers diluted 10 times in the analysis buffer, as well as one or several antigens at a concentration of 1 µg/mL. 50 µL of the tested solution were introduced in the dispositive. The dispositive was then rinsed by the addition of 40 µL of the analysis buffer for reducing the unspecific signal and thereby allowing a better reading of the immunochromatographic results.

As can be seen in fig.9A, 9B and 9C, for each assay the specific area corresponding to the introduced antigen is colored. Nevertheless, the coloration is slightly weaker when the tested solution contains all the antigens.

### Materials and Methods

### Material

Papers used for performing the above described experiments comprise celluloses CF1 and Chr1, as well as AE 98 Fast Nitrocellulose from Whatman (Maidstone, Kent, USA) and printing paper Xerox Premier 80 (Ref. 3R91720, Xerox, Norwalk, CT, USA). Immunochromatographic sticks were prepared using absorbent pad No. 470 from Schleicher and Schuell BioScience GmBH (Dassel, Germany) and plastic strips MIBA-020 backing card from Diagnostic Consulting Network (Carlsbad, USA). Materials were cut with the CM4000 Batch cutting system from BioDot (Irvine, USA).

Proteins (ovalbumin (OVA), Bovine Serum Albumin (BSA) and swine skin gelatin), as well as chemical products for preparing buffers were obtained from Sigma-Aldrich (St Louis, MO, USA). Water was purified by the Milli-Q system (Millipore, Brussels, Belgium).

Monoclonal murine antibodies were prepared at LERI (CEA, Saclay, France). Irradiations were conducted a room temperature in a U.V. chamber CN-15.LV UV viewing cabinet (Vilber Lourmat, Marne-la-Vallée, France). 96 wells plates (from Greiner Bio-One S.A.S. Division Bioscience, Les Ulis, France) were used as container for migrations on immunochromatographic strips. Colorimetric intensity resulting from colloidal gold was quantified with a Molecular imager VersaDoc MP4000, in association with the software Quantity One 1-D Analysis (Bio-Rad, Hercules, CA, USA).

### Methods

### Immunochromatographic assays.

Photoimmobilizations were performed as follows: a cellulose sheet was impregnated by a biomolecule solution; biomolecules (preferably antibodies) were concentrated by drying of the impregnated paper; the system was irradiated for inducing photoimmobilization; and intensive washing was performed for removing non-immobilized biomolecules.

For each membrane, an antibody solution OVA1 (murine antibody directed against OVA epitopes), at a concentration of 1mg/mL in potassium phosphate buffer 0.1M, pH 7.4, was poured on a 0.25 cm²-cellulose sheet (0.5 cm x 0.5 cm), at a rate of 40 µg/cm². Where appropriate, drying was performed at 37°C, in a ventilated oven, for 15 minutes. Irradiation was generally conducted at 365 nm (1050 µW/cm² at 365 nm, in visible light, the power characteristics of the lamp are not provided by the distributor). After irradiation, samples were rinsed with a phosphate buffer (potassium phosphate at 0.1M, pH 7.4) optionally enriched with salts and/or detergent (for instance potassium phosphate at 0.1M, pH 7.4 containing 0.5M NaCl and 0.5% (v/v) Tween 20). Salts allow the electrostatic interactions between biomolecules and surface to be limited, and the detergent reduces or prevents hydrophobic interactions. Salts and/or detergent thus contribute to restrain biomolecules adsorption.

Membranes were saturated with a gelatin solution (potassium phosphate at 0.1M, pH 7.4, NaCl 0.15M and gelatin 0.5% (w/v) for preventing non-specific binding on membranes. Saturation was performed overnight: membranes were impregnated with the gelatin solution at 4°C, then dried at 37°C in a ventilated oven for 30 minutes.

Immunochromatographic assays such as those implemented in the following examples were performed on immunochromatographic strips.

Typically, an immunochromatographic strip is composed of a loading area, a detection area and an absorbent pad, the whole being affixed onto a plastic carrier. The detection area is formed by antibody-grafted membranes. Migration is supported by two migration areas, surrounding the detection area, and composed of the same type of paper than the detection area, free of antibodies and saturated with gelatin. The immunoanalysis buffer, in the context of the present experiments, was composed of a potassium phosphate buffer at 0.1M, pH 7.4, containing BSA (0.1% (w/v)), salt (NaCl 0.15M) and detergent (Tween 20 0.5% (v/v)). The concentration of the OVA starting solution was of 10 µg/mL. Tracer antibodies were labelled with colloidal gold according to known methods (Kreich et al., Analytical Biochemistry 377 (2008) 182-188). Antibodies were diluted 10 times within the analysis buffer. Solutions to be analyzed were then dispatched in the wells of 96-well plates (100 µL/well) and strips were introduced in the wells. Migration was allowed for 15 minutes, then strips were dried at 37°C in a ventilated oven for 30 minutes. Finally, the colorimetric measure was performed right after rehydration of membranes with the analysis buffer.

The grafting rate and the activity rate were measured for each experimental condition.

The grafting rate of the cellulose paper was measured by establishing the difference between the antibody-grafted paper signal and the gelatin-grafted corresponding one displaying the unspecific adsorption of the goat-anti-mouse antibody labelled with colloidal gold ("tracer") onto the gelatin matrix.

The activity rate of the grafted antibodies was measured by establishing the difference between the signal obtained in presence and in absence of OVA (ovalbumin), for the binding of the OVA35 tracer (*i.e*., a colloidal-gold labelled antibody directed against OVA). Non-specific adsorption of the OVA35 tracer onto the antibody gelatin matrix was measured in absence of OVA.

Further, results also comprise a comparison with adsorption on cellulose only (negative control) and nitrocellulose (positive control). Considering that adsorption on nitrocellulose is the most frequently used method for immunochromatographic strips, it is herein considered as the reference and has been assimilated to 100% for both the grafting rate and the activity rate.

## Claims

1. A process for immobilizing biomolecules on a carrier comprising the steps of:
(i) impregnating the carrier with a solution containing the said biomolecules; and
(ii) irradiating the impregnated carrier resulting from step (i) with a light of a wavelength of at least 340 nm;
wherein said carrier is composed of cellulose, preferably of non-functionalized and/or chemical-free cellulose.

2. The process according to claim 1, further comprising a step of drying said impregnated carrier after step (i) and before step (ii).

3. The process according to any of the preceding claims, further comprising at least one step of washing the irradiated support resulting from step (ii).

4. The process according to any of the preceding claims wherein the irradiation light has a wavelength of from 340 nm to 800 nm, preferably of from 340 nm to 400 nm, preferably of from 400 nm to 800 nm, and is preferably of about 365 nm.

5. The process according to any of the preceding claims wherein the irradiation step (ii) is performed with a photoenergy of from 1 mJ/cm² to 500 J/cm², preferably of from 1 J/cm² to 80 J/cm², and is preferably of about 10 J/cm².

6. The process according to any of the preceding claims wherein the irradiation step (ii) is performed for a period of at least 1 second, preferably from 16 to 1280 minutes, preferably of about 160 minutes.

7. The process according to any of the preceding claims, wherein the carrier is selected in the form of a bead, well, sheet, powder, stick, plate, strip or as a tube, and is preferably in the form of a sheet.

8. The process according to any of the preceding claims, wherein said biomolecule is not functionalized and is selected from the group consisting of proteins or peptides, such as antibodies, antigens, enzymes, transcription factors, protein domains or binding proteins.

9. The process according to any of the preceding claims, wherein said biomolecule is displayed on the surface of a *bacteria,* a virus or a micro-organism or is free in solution.

10. The process according to any of the preceding claims wherein the said biomolecule is solubilized in a solvent selected in the group consisting of: water, phosphate buffer, carbonate buffer, borate buffer, HEPES buffer, MES buffer or any other aqueous biological buffer.

11. The process according to any of the preceding claims, wherein the washing buffer is selected from the group consisting of water, phosphate buffer, carbonate buffer, borate buffer, HEPES buffer, MES buffer or any other aqueous biological buffer, and further optionally comprises salts and/or detergent.

12. A process for immobilizing biomolecules on a carrier comprising the steps of :
(i) impregnating the carrier with a solution containing the said biomolecules;
(ii) drying the impregnated carrier resulting of step (i); and
(iii) irradiating the dried impregnated carrier resulting from step (ii) with a light of a wavelength of at least 340 nm;
wherein said carrier is composed of cellulose, preferably of non-functionalized and/or chemical-free cellulose; and
wherein the said irradiation step (iii) is performed with a photoenergy of at least 1 mJ/cm², preferably of at least 1 J/cm², and preferably of about 10 J/cm².

13. A grafted cellulose carrier obtained by the process of any of claims 1 to 12, wherein said cellulose carrier comprises biomolecules immobilized thereonto.

14. A bioassay device comprising at least one cellulose carrier according to claim 13 wherein said bioassay device is preferably an immunoassay device such as an immunochromatographic strip or an immunochromatographic multiplex system.

15. Use of at least one cellulose carrier according to claim 13 or of a bioassay device according to claim 14 for diagnosis, affinity chromatography, proteomics, genomics and/or drug screening.

16. A bioassay kit comprising at least one cellulose carrier according to claim 13 or at least one bioassay device according to claim 14.
